# EUROPÄISCHE PATENTANMELDUNG

(11) **EP 3 225 273 A1**
(43) Veröffentlichungstag der Anmeldung: **04.10.2017**
(21) Anmeldenummer: 17154142.8
(22) Anmeldetag: 01.02.2017
(51) Int. Cl.: A61M 16/10, B01D 53/00, B01D 53/22

(54) **VERFAHREN UND VORRICHTUNG ZUR SAUERSTOFFVERSORGUNG**

(30) Priorität: 30.03.2016 DE 102016003912
(71) Anmelder: Weinmann Emergency Medical Technology GmbH + Co. KG., 22525 Hamburg (DE)
(72) Erfinder: Die Erfindernennung liegt noch nicht vor
(74) Vertreter: Patentanwälte Klickow & Partner Partnerschaftsgesellschaft mbB

(57) **Zusammenfassung**

Die Erfindung betrifft ein Verfahren und sowie eine Vorrichtung zur Sauerstoffversorgung eines mobilen Beatmungsgerätes, bei dem wenigstens ein Sauerstoffkonzentrator zur Erzeugung eines Sauerstoffvolumenstromes sowie wenigstens ein Pufferspeicher zur Speicherung von Sauerstoffgas eingesetzt werden, wobei der wenigstens eine Sauerstoffkonzentrator und der wenigstens eine Pufferspeicher instationär betreibbar sind und funktionell derart zusammenwirken, dass die Energieversorgung des wenigstens einen Sauerstoffkonzentrators durch eine mobile Energieversorgung ermöglicht ist.

## Beschreibung

Die Erfindung betrifft ein Verfahren und sowie eine Vorrichtung zur Sauerstoffversorgung eines mobilen Beatmungsgerätes, bei dem wenigstens ein Sauerstoff-konzentrator zur Erzeugung eines Sauerstoffvolumenstromes sowie wenigstens einen Pufferspeicher zur Speicherung von Sauerstoffgas eingesetzt werden.

In der modernen Medizin werden in einer Vielzahl unterschiedlicher Situationen Medikamente verschiedenster Art eingesetzt, um akute und chronische Symptome verursacht durch Krankheiten, schädliche äußere Einwirkungen und unfallbedingte Schäden zu lindern oder zu beseitigen. Auch in Notfallsituationen werden Medikamente eingesetzt, um beispielsweise drohendes Versagen der Vitalfunktionen eines Unfallopfers oder krankheitsbedingtes Kollabieren von Personen zu verhindern.

In Notfallsituationen, insbesondere bei ambulanten Erstversorgungen von Patienten, ist es häufig erforderlich, dass Sauerstoff über eine Notfallbeatmung verabreicht wird. Um diese mit Sauerstoff angereicherte Atemluft dem Patienten vor Ort zuführen zu können, werden mobile und ggfs. auch tragbare, in der Regel pneumatisch arbeitende Beatmungsgeräte beispielsweise in Rettungsfahrzeugen oder Notarztwagen mitgeführt, um an einem Notfallort verfügbar zu sein.

Um die sauerstoffangereicherte Atemluft durch das Beatmungsgerät, das häufig auch als Respirator bezeichnet wird, dem Patienten zur Verfügung stellen zu können, muss die Atemluft beziehungsweise der benötigte Atemluftvolumenstrom mit Sauerstoff angereichert werden, um auf diese Weise die Sauerstoffkonzentration auf das medizinisch erforderliche Maß zu erhöhen.

Die Sauerstoffanreicherung wird üblicherweise durch Zuführung von höher- oder hochkonzentriertem Sauerstoff erreicht, die der durch das Beatmungsgerät bereitgestellten Luft beigemischt wird, um auf diese Weise die sauerstoffangereicherte Atemluft zu erzeugen. Um die Zuführung von höher- oder hochkonzentriertem Sauerstoff zu realisieren, werden üblicherweise entweder Speichermedien zur Sauerstoffbevorratung oder Vorrichtungen zur Sauerstoffkonzentration eingesetzt. Alternativ zur Sauerstoffzuführung zum Beatmungsgerät können auch geeignete Vorrichtungen zur Sauerstoffkonzentration integrativer Bestandteil des Beatmungsgerätes sein.

Bekannte Vorrichtungen zur Sauerstoffkonzentration sind beispielsweise Sauerstoffanreicherungsgeräte, die auch als Sauerstoffkonzentratoren bezeichnet werden. In diesen Sauerstoffkonzentratoren wird Stickstoff aus der Luft herausgefiltert und dadurch Sauerstoff mit einer Konzentration von etwa 93% bereitgestellt. Hierzu muss Luft komprimiert und Adsorptionsfiltern zugeführt werden, die dann der Luft den Stickstoff entziehen.

Pneumatische Sauerstoffkonzentratoren mit Adsorptionsfilterbasis arbeiten vielfach nach dem Druckhub-Adsorptionsprinzip und umfassen primär die Funktionsschritte
a) Druckluftzuführung in einen Zylinder, der eine Adsorptionssäule eines Adsorbens aufweist, das selektiv Stickstoff adsorbiert,
b) Fortsetzen der Druckluftzuführung in den Zylinder derart, dass durch die Druckluftsäule eine Zone des adsorbierten Stickstoffgases durch den Zylinder in Längsrichtung verschoben wird,
c) Aufnehmen des aus dem Zylinder strömenden Sauerstoffgases,
d) Stoppen der Druckluftzufuhr in den Zylinder,
e) Abschließen der Aufnahme von Sauerstoff aus dem Zylinder,
f) Entladen eines Teils des unter Druck stehenden Gases, das in dem Zylinder verbleibt, und
g) Zurückbringen eines Teils des Sauerstoffs in den Zylinders, um durch die Säule in die entgegen gesetzte Richtung zu strömen, um das Gas (Stickstoff) zu desorbieren und um das desorbierte Gas (Stickstoff) aus dem Zylinder abzuführen.

Somit basiert das Druckhub-Adsorptionsprinzip auf dem Wirkungszusammenhang, dass durch teilweises oder nahezu vollständiges Entfernen des Stickstoffes aus der Umgebungsluft, bestehend aus einer Sauerstoff-Stickstoffmischung, die Sauerstoffkonzentration angehoben wird.

Andere Verfahren zur Erhöhung einer Sauerstoffkonzentration basieren beispielsweise auf besonderen Sauerstofftransporteigenschaften spezieller Materialien. Beispielsweise können keramische Werkstoffe mit einer besonderen Zusammensetzung und Gitterstruktur bei höheren Temperaturen die Fähigkeit zur Sauerstoffleitung aufweisen. Durch einen unterschiedlichen Sauerstoffpartialdruck in zwei Räumen getrennt durch eine Keramik in Form einer Sauerstoff-Transport-Membran mit geeigneten Sauerstofftransporteigenschaften kann einseitig das Sauerstoffpotential erhöht werden. Verfahren basierend auf permeablen Membranen separieren Sauerstoff durch mechanische Einwirkung.

Ein anderes gleichfalls mechanisch basierendes Gewinnungsprinzip kann realisiert werden durch Molekularsiebe. Siebe dieser Art sind häufig durch natürliche und synthetische Zeolithe oder Kohlenstoffverbindungen mit hoher Adsorptionskapazität für Gase, Dämpfe und gelöste Stoffe mit bestimmten Molekülgrößen aufgebaut. Je nach Porengröße können Molekularsiebe Stickstoff aufnehmen und beispielsweise über Druckwechsel-Adsorption (vgl. Ausführungen oben) sauerstoffkonzentrierend oder über CMS (Carbo Molecular Sieves) stickstofferhöhend wirken.

Während bei stationären Beatmungsgeräten, beispielsweise im Einsatzbereich der Intensivmedizin, alle zuvor genannten Formen zur Bereitstellung von sauerstoffangereicherter Atemluft Anwendung finden, ergeben sich bei instationär und damit mobil verwendeten Beatmungsgeräten mit dem Kriterium der Mobilität, insbesondere von Notfallbeatmungsgeräten, besondere Anforderungen und Probleme.

Viele der mobilen Sauerstoffversorungslösungen für instationäre Beatmungsgeräte sehen die Bereitstellung des Sauerstoffes in Form von Speichermedien vor, vorzugsweise in Flaschenform und meist als Druck- oder Hochdruckflaschen. Die Verfügbarkeit von höher- und hochkonzentriertem Sauerstoff in Flaschenspeichermedien ist über die Ländergrenzen hinweg weit verbreitet, aber nicht überall lückenlos verfügbar. Je nach Region oder im Katastrophenfall ist die Flaschenversorgung nicht gesichert, sodass gespeicherter Sauerstoff nicht uneingeschränkt zur Verfügung steht.

Im Rahmen mobiler Sauerstoffversorgungslösungen werden in besonderen Einzelfällen auch Sauerstoffkonzentratoren eingesetzt. Problematisch dabei ist jedoch, dass erheblich Mengen vorzugsweise elektrischer Energie zum Betrieb dieser Vorrichtungen erforderlich sind.

Bei einem Sauerstoffvolumenstrom von ca. 5 Umin benötigen Sauerstoff-konzentratoren unabhängig vom verwendeten Konzentrationsverfahren etwa 300 W elektrische Leistung. Um Sauerstoffkonzentratoren anstelle von sauerstoffbevorratenden Flaschen einsetzen zu können, müssen diese im Mittel bis zu 15 Umin abgeben können, um die Versorgung des Beatmungsgerätes in ausreichender Menge sicherzustellen. Damit wären Sauerstoffkonzentratoren erforderlich, die etwa die dreifache Größe als die handelsüblicher Geräte aufweisen und eine elektrische Leistung in der Größenordnung von 1 kW aufnehmen.

Beispielsweise in Rettungsfahrzeugen oder Notarztwagen ist die Energiebereitstellung dieser Größenordnungen nicht oder nur eingeschränkt durch Energiespeicher beispielsweise in Form von Akkumulatoren möglich. Durch den hohen Leistungsbedarf sind Vorrichtungen für die Sauerstoffkonzentration innerhalb mobiler und direkter Sauerstoffversorgungen daher wenig geeignet.

Weiterhin muss für den Betrieb eines Notfallbeatmungsgerätes ein Sauerstoffdruck von mindestens 3 bar zur Verfügung stehen. Ebenso sind Spitzenvolumenströme von mindestens 100 Umin in Einzelfällen erforderlich, damit Sauerstoffkonzentratoren anstelle von Sauerstoffbevorratungsflaschen zur Versorgung der mobilen Beatmungsgeräte eingesetzt werden können. Das können handelsübliche Sauerstoffkonzentratoren im Normalfall nicht leisten.

Es ist daher Aufgabe der Erfindung, eine Sauerstoffzufuhr für instationäre Beatmungsgeräte bereitzustellen, die insbesondere die mobile Sauerstoffversorgung unterstützt.

Zur Lösung dieser Aufgabe schlägt die Erfindung ein Verfahren und sowie eine Vorrichtung zur Sauerstoffversorgung eines mobilen Beatmungsgerätes vor, bei dem wenigstens ein Sauerstoffkonzentrator zur Erzeugung eines Sauerstoffvolumenstromes sowie wenigstens einen Pufferspeicher zur Speicherung von Sauerstoffgas eingesetzt werden, wobei der wenigstens eine Sauerstoffkonzentrator und der wenigstens eine Pufferspeicher instationär betreibbar sind und funktionell derart zusammenwirken, dass die Energieversorgung des wenigstens einen Sauerstoffkonzentrators durch eine mobile Energieversorgung ermöglicht ist.

Anstelle der Flaschenbereitstellung hochkonzentrierten, druckbeaufschlagten Sauerstoffes für das Beatmungsgerät, wird erfindungsgemäß durch einen Sauerstoffkonzentrator mit handelsüblichen Leistungsaufnahmewerten von ca. 300 W und realisierbaren Sauerstoffvolumenströmen von ca. 5 Umin Sauerstoff erzeugt, der dann einer Druckerhöhungseinrichtung zugeführt und durch diese in einen druckbeaufschlagten Pufferspeicher eingebracht wird. Die erfindungsgemäße Vorrichtung kann kontinuierlich oder diskontinuierlich, in jedem Fall aber durch den Pufferspeicher zeitlich unabhängig von dem aktuellen Sauerstoffbedarf betrieben werden.

Durch die Verwendung eines mobilen Sauerstoffkonzentrators in Kombination mit einem Sauerstoffbevorratungsspeicher ergeben sich mobile Anwendungsgebiete, beispielsweise innerhalb von Rettungswagen. Durch die Kombination eines Sauerstoffkonzentrators mit einem Sauerstoffbevorratungsspeicher kann ein handelsüblicher Sauerstoffkonzentrator mit entsprechendem Energieaufnahmebedarf realisiert werden. Dem physikalischen Grundprinzip der Leistung gleich Arbeit pro Zeiteinheit folgend wird der Zeitfaktor durch den Pufferspeicher von dem relativ kurzen Bedarfszeitraum auf ein Zeitfenster zur Befüllung des Speichers hin ausgedehnt und damit die Spitzenleistungsaufnahme signifikant reduziert.

Die Erfindung erkennt, dass die Größendimensionierung des Sauerstoffbevorratungsspeichers von dem Sauerstoffvolumenstrom abhängig ist, den der Sauerstoffkonzentrator zur Verfügung stellt. Beispielsweise können bereits ein oder zwei 10 Liter Sauerstoffbevorratungsspeicher ausreichend sein, wenn ein Sauerstoffvolumenstrom von 0,3 bis 0,5 Liter/min vorliegt.

Durch die Vorrichtung, bestehend aus Sauerstoffkonzentrator und nachgeschaltetem Pufferspeicher, sowie eine diesen Komponenten zwischengeschaltete Druckerhöhungseinrichtung sind Beatmungsvorgänge mit sauerstoffangereicherter Atemluft in und in unmittelbarer Nähe zu einem Rettungswagen, Notfalleinsatzwagen oder Notfallarztwagen möglich.

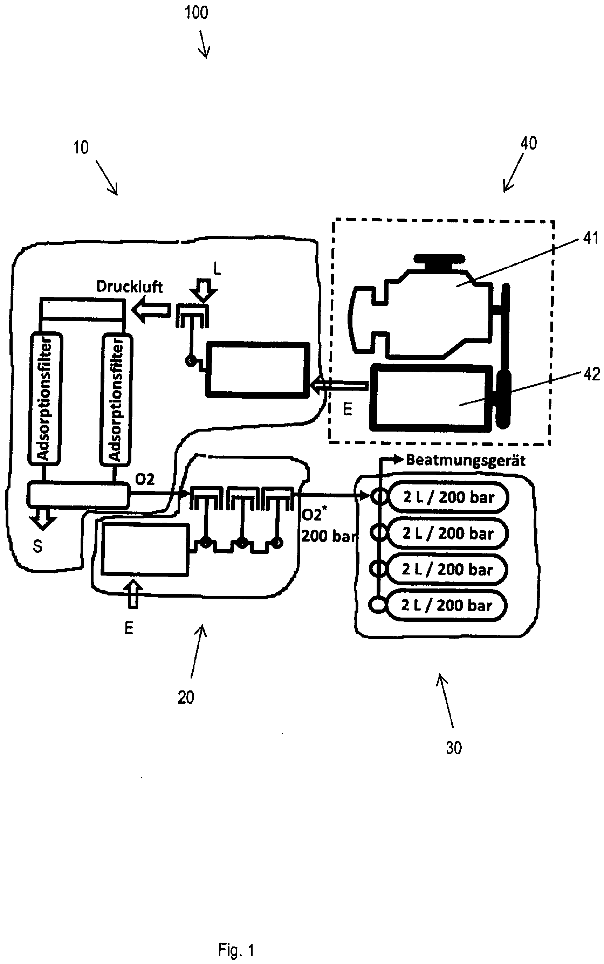

Das erfindungsgemäße Grundprinzip wird nachfolgend an der Funktionsskizze dargestellt in Figur 1 erläutert. Gezeigt ist die Kombination verschiedener Vorrichtungskomponenten mit ihren jeweils interaktiven Wirkungszusammenhängen zueinander und der Gesamtfunktion zur Realisierung der Sauerstoffversorgung eines mobilen Beatmungsgerätes für die Versorgung eines Subjektes mit sauerstoffangereicherter Atemluft.

Die Vorrichtung zur Sauerstoffversorgung (100) besteht im Wesentlichen aus wenigstens einem Sauerstoffkonzentrator (10), dem optional wenigstens eine Druckerhöhungseinrichtung (20) zur Druckbeaufschlagung des konzentrierten Sauerstoffgases (O2) nachgeschaltet ist und aus wenigstens einem Pufferspeicher (30) zur Aufnahme und/oder Speicherung bzw. Pufferung des erzeugten Sauerstoffgases (O2*).

Im gezeigten Ausführungsbeispiel ist der Sauerstoffkonzentrator (10) auf dem Druckhub-Adsorptionsprinzip basierend ausgeführt. Realisierbar sind auch andere als auf diesem Wirkprinzip basierende Sauerstoffkonzentratoren. Unabhängig vom Wirkprinzip des Konzentrators muss gewährleistet sein, dass der eingesetzte Sauerstoffkonzentrator (10) durch eine mobile Energiebereitstellung (40), die beispielsweise in einem Rettungswagen mitgeführt werden kann oder Bestandteil eines Fahrzeugs ist, mit Energie (E) im erforderlichen Umfang versorgt werden kann.

Die mobile Energiebereitstellung (40) kann gebildet sein aus wenigstens einem Energieerzeuger und/oder wenigstens einem Energiespeicher. In der Praxis ist elektrische Energie zur Versorgung des Sauerstoffkonzentrators (10) vorteilhaft, sodass die mobile Energiebereitstellung (40) beispielsweise aus dem Antriebsmotor des Fahrzeugs mit nachgeschaltetem elektrischem Generator gebildet sein kann. Ergänzend oder alternativ kann ein elektrischer Energiespeicher, beispielsweise Akkumulator, eingesetzt sein. Anstelle des elektrischen Generators und in Abhängigkeit vom energetischen Wirkprinzip des Sauerstoffkonzentrators (10) sind auch Kompressoren, Fluidpumpen etc. als Energiequellen bzw. Energiemedien möglich.

Wird eine mobile Energiebereitstellung (40) als Kombination aus Energieerzeuger und Energiespeicher realisiert, kann der Energiespeicher verschiedene Funktionen erfüllen, insbesondere die zeitbegrenzte alleinige Energieversorgung, die Energieniveaustabilisierung bei instabiler oder ungleichmäßiger Energieerzeugung sowie Energielinearisierung zur Versorgung der jeweils nachgeschalteten Aggregate.

Infolge der begrenzten Energiekapazität der mobilen Energiebereitstellung (40) kann ein Sauerstoffkonzentrator (10) üblicherweise mit einer Sauerstoffgasausgangsleistung in Form eines Sauerstoffvolumenstroms (O2) von bis zu ca. 5 Umin realisiert werden. Sauerstoffkonzentratoren (10) mit höheren Sauerstoffgasausgangsleistungen sind vielfach wirtschaftlich sinnvoll in mobilen Anwendungen nicht betreibbar, da deren Energieaufnahme für mobile Energiequellen in Rettungsfahrzeugen oder Notarztwagen zu hoch ist.

Infolge der durch die wirtschaftlich und räumlich begrenzten Möglichkeiten der Energiebereitstellung für entsprechende Sauerstoffkonzentratoren (10) in mobilen Anwendungsfällen ergibt sich ein praktisch sinnvoller Sauerstoffvolumenstrom (O2) von ca. 5 Umin, der üblicherweise bereitgestellt wird, im Niederdruckbereich. Da diese Sauerstoffausgangsleistung für die Versorgung des Beatmungsgerätes in der Regel nicht ausreichend ist, sieht die Erfindung vor, dass dem wenigstens einen Sauerstoffkonzentrator (10) vorzugsweise wenigstens eine Druckerhöhungseinrichtung (20) zur Druckbeaufschlagung des konzentrierten Sauerstoffgases (O2) nachgeschaltet ist und dass wenigstens ein Pufferspeicher (30) zur Aufnahme und/oder Speicherung bzw. Pufferung des druckbeaufschlagten Sauerstoffgases (O2*) verwendet wird.

Auf die wenigstens eine Druckerhöhungseinrichtung (20) kann verzichtet werden, wenn der Pufferspeicher (30) den konzentrierten Sauerstoff (O2) im Niederdruckbereich bzw. im Rahmen des Ausgangsdruckes des Sauerstoffkonzentrators (10) von üblicherweise ca. 0,8 bar zu speichern vermag. Solche Niederdruck-Pufferspeicher (30) müssen, um entsprechende Sauerstoffvolumina aufnehmen zu können, räumlich deutlich größer ausgeführt sein. Um den begrenzten Platzverhältnisse in Rettungs- oder Notarztwagen oder mobilen Trageinrichtungen Rechnung zu tragen ist, bevorzugt ein Pufferspeicher (30) in Hochdruckbauweise mit Druckverhältnissen von bis zu 200 bar einzusetzen.

Der erfindungsgemäße Pufferspeicher (30) in vorzugsweise Hochdruckspeichervariante kann aus wenigstens einem beliebig geformten Druckbehälter bestehen. Besonders vorteilhaft sind wenigstens ein Druckbehälter in Flaschenform mit einem Volumina von 2 Litern und ausgelegt für Druckverhältnisse von bis zu 200 bar Überdruck. Um die Kapazität des Pufferspeichers (30) anzupassen bzw. variabel zu gestalten können eine oder eine Mehrzahl von Druckflaschen mit gleichen oder unterschiedlichen Volumina in Reihe oder parallel geschaltet werden.

Neben der Überwindung der in mobilen Systemen vorliegenden begrenzten Energieversorgungssituationen durch die vorgeschlagene Vorrichtung zur Sauerstoffversorgung (100) beschäftigt sich die erfindungsgemäße Lehre im Rahmen der gegebenen räumlichen Verhältnisse zum Beispiel in Rettungswagen auch mit der Verortung der Vorrichtungskomponenten in solchen Fahrzeugen und schlägt als eine von mehreren Möglichkeiten vor, dass der Sauerstoffkonzentrator (10) im Motorraum des Fahrzeugs angeordnet wird.

Da die anzusaugende Luft (L) sauber und frei von Verunreinigungen sein muss, ist in diesem Fall bevorzugt daran gedacht, Luft nicht aus dem schmutzbelasteten Umfeld des Motorraumes anzusaugen, sondern über einen im Fahrzeug verlegten Schlauch, dessen Mündungsende derart positioniert ist, dass schmutzarme Luft ansaugbar ist.

Die Anordnung des Sauerstoffkonzentrators (10) im Motorraum des Fahrzeugs ist vorteilhaft, weil die Wellenleistung des Fahrzeugmotors direkt abgegriffen werden kann und weil die Geräusch- und Wärmeentwicklung innerhalb des durch die Karosserie wenigstens teilweise eingehausten Motorraumes verbleibt und die Transport- und Ladefläche des Fahrzeugs nicht beansprucht wird.

Wird die mobile Energieversorgung (E) in elektrischer Form realisiert, ist daran gedacht, die elektrische Ausrüstung des Rettungs- oder Notarztfahrzeugs zu nutzen. Üblicherweise verfügen Fahrzeuge dieser Art über eine mit dem Verbrennungsmotor zusammenwirkende Lichtmaschine sowie einen Energiespeicher in Form eines Akkumulators. Ergänzend oder stattdessen kann ein separater Generator durch den Antriebsmotor des Fahrzeugs angetrieben werden, der optional mit dem Fahrzeugakkumulator und/oder separaten Speichereinrichtungen zusammenwirkt.

Weiterhin ist daran gedacht, die mobile Energieversorgung temporär durch eine stationäre Energieversorgung zu ergänzen. Die Erfindung macht sich die Beobachtung zu Nutze, dass der erforderliche Sauerstoffvolumenstrom zur Versorgung des Beatmungsgerätes über einen betrachteten Zeitraum diskontinuierlich und relativ gesehen nur kurzzeitig realisiert werden muss. Ähnlich und infolge des Pufferspeichers (30) zeitpunkt- und umfangsversetzt trifft dies auch auf die benötigte Antriebsenergie (E) der jeweiligen Vorrichtungskomponenten zur Sauerstoffversorgung (100) zu.

Erfindungsgemäß ist daher in Zeitphasen außerhalb von Einsatzfahrten vorgesehen, die Rettungs- und Notarztfahrzeuge mit einer externen Energiequelle zu verbinden und auf diese Weise Energie zum Betrieb des Sauerstoffkonzentrators (10) und der wenigstens einen Druckerhöhungseinrichtung (20) zur Befüllung des Pufferspeichers (30) zuzuführen.

Gemäß einer bevorzugten Ausführungsform ist insbesondere daran gedacht, dass die Druckerhöhungseinrichtung (20) durch elektrische und/oder pneumatische und/oder fluidische und/oder eine Wellenleistung mit Energie (E) versorgbar ist.

Gemäß einer bevorzugten Ausführungsform ist insbesondere daran gedacht, dass die mobile Energieversorgung (40) aus wenigstens einem Energieerzeuger und/oder einem Energiespeicher gebildet ist.

Gemäß einer bevorzugten Ausführungsform ist insbesondere daran gedacht, dass die mobile Energieversorgung (40) integraler Bestandteil eines Fahrzeugs ist.

Gemäß einer bevorzugten Ausführungsform ist insbesondere daran gedacht, dass die mobile Energieversorgung (40) aus wenigstens einem Antriebsaggregat (41) und einem elektrischen und/oder pneumatischen und/oder einem fluidischen Energieerzeuger (42) gebildet ist.

Gemäß einer bevorzugten Ausführungsform ist insbesondere daran gedacht, dass der wenigstens eine Energieerzeuger (42) und/oder der Sauerstoffkonzentrator (10) und/oder die Druckerhöhungseinrichtung (20) im Motorraum eines Fahrzeugs angeordnet ist.

## Patentansprüche

1. Vorrichtung zur Sauerstoffversorgung (100) eines mobilen Beatmungsgerätes, aufweisend wenigstens einen Sauerstoffkonzentrator (10) zur Erzeugung eines Sauerstoffvolumenstromes (O2) sowie wenigstens einen Pufferspeicher (30) zur Speicherung von Sauerstoffgas, **dadurch gekennzeichnet, dass** der wenigstens eine Sauerstoffkonzentrator (10) und der wenigstens eine Pufferspeicher (30) instationär betreibbar sind und funktionell derart zusammenwirken, dass die Energieversorgung des wenigstens einen Sauerstoff-konzentrators (10) durch eine mobile Energieversorgung (40) ermöglicht ist.

2. Vorrichtung zur Sauerstoffversorgung (100) nach Anspruch 1, **dadurch gekennzeichnet, dass** wenigstens eine Druckerhöhungseinrichtung (20) dem wenigstens einen Sauerstoffkonzentrator (10) und dem Pufferspeicher (30) zwischengeschaltet ist, sodass eine Druckerhöhung des Sauerstoffvolumenstroms (O2) ausgangs des Sauerstoffkonzentrators (10) unterstützt ist.

3. Vorrichtung zur Sauerstoffversorgung (100) nach Anspruch 2, **dadurch gekennzeichnet, dass** die Druckerhöhung des Sauerstoffvolumenstromes (O2*) auf 200 bar unterstützt ist.

4. Vorrichtung zur Sauerstoffversorgung (100) nach Anspruch 1, **dadurch gekennzeichnet, dass** der Pufferspeicher (30) als Hochdruckpufferspeicher für Druckverhältnisse bis zu 200 bar ausgeführt ist.

5. Vorrichtung zur Sauerstoffversorgung (100) nach Anspruch 1, **dadurch gekennzeichnet, dass** der Pufferspeicher (30) einen Sauerstoffentnahmevolumenstrom von im Mittel 15 Umin und im Maximum von bis zu 100 Umin unterstützt.

6. Vorrichtung zur Sauerstoffversorgung (100) nach Anspruch 1, **dadurch gekennzeichnet, dass** der Pufferspeicher (30) aus einer Mehrzahl von Speicherbehältern mit gleichen oder unterschiedlichen Volumina in reihe und/oder parallel geschaltet gebildet ist.

7. Vorrichtung zur Sauerstoffversorgung (100) nach Anspruch 1, **dadurch gekennzeichnet, dass** der Sauerstoffkonzentrator (10) ein pneumatisch auf dem Druckhub-Adsorptionsprinzip basierend arbeitender Konzentrator ist.

8. Vorrichtung zur Sauerstoffversorgung (100) nach Anspruch 1, **dadurch gekennzeichnet, dass** der Sauerstoffkonzentrator (10) ein mechanisch basierendes Verfahren mit einer Sauerstoff-Transportmembran umfasst.

9. Vorrichtung zur Sauerstoffversorgung (100) nach Anspruch 1, **dadurch gekennzeichnet, dass** der Sauerstoffkonzentrator (10) ein mechanisch basierendes Verfahren mit einem Molekularsieb umfasst.

10. Vorrichtung zur Sauerstoffversorgung (100) nach Anspruch 1, **dadurch gekennzeichnet, dass** der Sauerstoffkonzentrator (10) einen Sauerstoffvolumenstrom von etwa 5 Umin bei einem Ausgangsdruck von etwa 0,8 bar erzeugt und eine Leistung von etwa 300 W aufnimmt.

11. Vorrichtung zur Sauerstoffversorgung (100) nach Anspruch 1, **dadurch gekennzeichnet, dass** der Sauerstoffkonzentrator (10) durch elektrische und/oder pneumatische und/oder fluidische und/oder eine Wellenleistung mit Energie (E) versorgbar ist.

12. Verfahren zur Sauerstoffversorgung eines mobilen Beatmungsgerätes unter Verwendung einer Vorrichtung (100) nach einem der Ansprüche 1 bis 11, aufweisend die Schritte
a) kontinuierliche oder diskontinuierliche Erzeugung eines Sauerstoffvolumenstromes (O2) durch wenigstens einen instationär arbeitenden Sauerstoffkonzentrator (10),
b) Zuführung des durch den Sauerstoffkonzentrators (10) erzeugten Sauerstoffs in einen Pufferspeicher (30) zur zeitdiskreten Sauerstoffversorgung eines Beatmungsgerätes,
**dadurch gekennzeichnet, dass** die zeitdiskrete Entnahme von Sauerstoff aus dem Pufferspeicher (30) temporär kleiner als die Zuführung eines durch den instationär arbeitenden Sauerstoffkonzentrator (10) erzeugten Sauerstoffvolumenstromes ist, sodass die Energieversorgung des wenigstens einen Sauerstoffkonzentrators (10) durch eine mobile Energieversorgung (40) unterstützt ist.

13. Verfahren zur Sauerstoffversorgung eines mobilen Beatmungsgerätes nach Anspruch 12, **dadurch gekennzeichnet, dass** die Erzeugung eines Sauerstoffvolumenstromes (O2) durch wenigstens einen instationär arbeitenden Sauerstoffkonzentrator (10) durch die Pufferung von Sauerstoffgas in dem Pufferspeicher (30) zeitlich unabhängig von dem aktuellen Sauerstoffbedarf des mobilen Beatmungsgerätes erfolgt.

14. Verfahren zur Sauerstoffversorgung eines mobilen Beatmungsgerätes nach Anspruch 12, **dadurch gekennzeichnet, dass** die mobile Energieversorgung (40) temporär durch eine stationäre Energieversorgung ergänzt oder ersetzt wird.

15. Verfahren zur Sauerstoffversorgung eines mobilen Beatmungsgerätes nach Anspruch 12, **dadurch gekennzeichnet, dass** die Sauerstoffzufuhr (O2, O2*) zum wenigstens einen Pufferspeicher (30) temporär durch einen stationär arbeitenden Sauerstoffkonzentrator realisiert ist.
